(19) Europäisches Patentamt / European Patent Office / Office européen des brevets

(11) **EP 3 865 855 A1**

(12) **EUROPÄISCHE PATENTANMELDUNG**

(43) Veröffentlichungstag:
**18.08.2021 Patentblatt 2021/33**

(21) Anmeldenummer: **21151863.4**

(22) Anmeldetag: **15.01.2021**

(51) Int Cl.:
**G01N 21/27** (2006.01)   **G01N 21/31** (2006.01)
**G01N 21/84** (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **12.02.2020 DE 102020000904**

(71) Anmelder: **Deere & Company**
**Moline, IL 61265 (US)**

(72) Erfinder:
• **Schade, Dr. Peter**
  **68163 Mannheim (DE)**
• **Klein, Helge**
  **68163 Mannheim (DE)**
• **Weber, Frank**
  **68163 Mannheim (DE)**

(74) Vertreter: **Holst, Sönke**
**John Deere GmbH & Co. KG**
**Global Intellectual Property Services**
**John-Deere-Strasse 70**
**68163 Mannheim (DE)**

(54) **NAHINFRAROT-SPEKTROMETERANORDNUNG FÜR EINE LANDWIRTSCHAFTLICHE ARBEITSMASCHINE**

(57) Eine Spektrometeranordnung (10) für eine landwirtschaftliche Arbeitsmaschine (40, 130) umfasst:
eine Lichtquelle (16) zur Beleuchtung einer zu untersuchenden Probe (12),
ein Spektrometer (18) zur Bereitstellung eines Spektralsignals (20) hinsichtlich der wellenlängenspezifischen Intensität von der Probe (12) reflektierten und/oder transmittiertem Lichts, und
einer Auswertungseinrichtung (24), die konfiguriert ist, anhand des Spektralsignals (22) des Spektrometers (18)
und von abgespeicherten Kalibrierdaten den Gehalt der Probe (12) an einem oder mehreren Inhaltsstoffen zu bestimmen,
wobei der Auswertungseinrichtung (24) ein Signal (26) hinsichtlich einer Eigenschaft der Probe (12) zuführbar ist und die Auswertungseinrichtung (24) konfiguriert ist, das Signal (26) bei der Bestimmung des Gehalts der Probe (12) an dem einen oder mehreren Inhaltsstoffen zu berücksichtigen.

FIG. 1

EP 3 865 855 A1

## Beschreibung

Die Erfindung betrifft eine Spektrometeranordnung für eine landwirtschaftliche Arbeitsmaschine, umfassend:

eine Lichtquelle zur Beleuchtung einer zu untersuchenden Probe,
ein Spektrometer zur Bereitstellung eines Spektralsignals hinsichtlich der wellenlängenspezifischen Intensität von der Probe reflektierten und/oder transmittiertem Lichts, und
einer Auswertungseinrichtung, die konfiguriert ist, anhand des Spektralsignals des Spektrometers und von abgespeicherten Kalibrierdaten den Gehalt der Probe an einem oder mehreren Inhaltsstoffen zu bestimmen.

### Stand der Technik

Die Messung von Inhaltsstoffen mittels optischer Spektroskopie, insbesondere Nah-Infrarot Spektroskopie, ist heute eine etablierte Technologie. Sie wird in vielen Anwendungen, beispielsweise in der Lebensmittelindustrie und in der Landwirtschaft genutzt, z.B. zur Untersuchung des Zustands auf einem Feld stehender Pflanzen, von Inhaltsstoffen geernteter Pflanzenteile oder der Zusammensetzung von Gülle.

Die Vorhersage der Inhaltsstoffwerte erfolgt in der Regel durch Anwendung eines linearen Modells in der Form *Inhaltsstoff* = $\Sigma_i$ *Spektrum* ∗ *Koeffizienten* + *Offset.* Es werden somit die Intensitäten der Bestandteile i des Spektrums mit dem Wert i zugehörigen Koeffizienten multipliziert und aufaddiert. Die Koeffizienten und der Offset werden anhand eines Lerndatensatzes aus Spektren mit zugehörigen Metadaten, den unabhängig von der spektroskopischen Messung im Labor bestimmten Inhaltsstoffwerten, durch geeignete lineare Regressionsmethoden (Kalibrieralgorithmus) festgelegt. Es erfolgt somit an einer Anzahl von Proben mit jeweils bekannten Inhaltsstoffanteilen eine spektroskopische Messung, um für einen zu untersuchenden Inhaltsstoff für jede der i untersuchten Wellenlängen den zugehörigen Koeffizienten und den Offset zu bestimmen. Diese Kalibrierdaten werden schließlich abgespeichert und bei einer anschließenden Messung einer unbekannten Probe genutzt, um anhand des aufgenommenen Spektrums den Anteil des Inhaltsstoffs zu ermitteln (s. J.B. Reeves III et al., Near-Infrared Spectroscopic Determination of Carbon,Total Nitrogen, and Ammonium-N in Dairy Manures, J Dairy Sci 83 (2000), Seiten 1829-1836).

Bei vielen Inhaltsstoffen haben die Temperatur und andere Parameter, z.B. der Druck, bei der Messung einen Einfluss auf das gemessene Spektrum, beispielsweise bei Flüssigkeiten wie Gülle. Ein Ansatz zur Lösung dieses Problems besteht darin, in den Lerndatensatz Proben mit verschiedenen Temperaturen aufzunehmen (vgl. beispielsweise C. Paul et al., Influence of sample temperature on the assessment of quality characteristics in undried forages by Near Infrared Spectroscopy (NIRS), Landbauforschung Völkenrode 4/2002, Seiten 229-237, A. Peinado et al., Temperature-induced variation for NIR tensor-based calibration, Chemometrics and Intelligent Laboratory Systems 83 (2006), Seiten 75-82 oder WO 2005/083386 A1). Hierbei 'ermittelt' der Kalibrieralgorithmus nur die durch Veränderung der Inhaltsstoffe bewirkten Veränderungen der Spektren, da die durch Temperaturveränderung bewirkten spektralen Veränderungen nicht mit einer Veränderung des Inhaltsstoffs korrelieren.

Ein solcher Ansatz ist für ein lineares Kalibrationsmodell die naheliegendste Möglichkeit, das Kalibrationsmodell gegenüber Temperatureinflüssen robust zu machen. Der Nachteil dieses Verfahrens besteht in einem Verlust an Sensitivität (Genauigkeit), da die spektralen Veränderungen die durch die Randparameter (Temperatur, Druck, ...) hervorgerufen werden, die Veränderungen, die durch die Variation der Inhaltsstoffe hervorgerufen werden, verdecken können. Dennoch handelt es sich hierbei um die in der Landwirtschaft üblicherweise angewandte Vorgehensweise, zumal sie keine Erfassung der Temperatur der Probe oder anderer Randparameter erfordert. Die Auswirkungen der zu erwartenden Variationen der Temperatur sind bereits im (hinsichtlich der Temperaturvariationen dotierten) Kalibriermodell enthalten, wenn auch auf Kosten der Genauigkeit.

Eine andere Vorgehensweise besteht darin, eine Temperaturkompensation im Referenzwertraum mittels einer Kalibriertransfermethode durchzuführen. Durch eine Anpassung der Vorhersagewerte auf Basis der linearen Regression können die bei einer anderen als bei der Referenzierung verwendeten Temperatur gemessenen Werte auf die bei der Referenztemperatur gemessenen Referenzwerte zurücktransformiert werden ("Bias and Skew adjustment", auch als a *posteriori*-Korrektur bezeichnet). Hier wird eine lineare Gleichung angewandt, um die aus den Spektren abgeleiteten Inhaltsstoffanteile temperaturabhängig zu korrigieren. Ein weiterer Ansatz sieht vor, den kompletten Spektrensatz im Spektraldatenraum derart zu verschieben, dass die Spektren im Bereich der bei der jeweiligen Messtemperatur zu erwartenden Spektren liegen (auch als a priori-Korrektur bezeichnet). Geometrisch gesprochen wird dieser Ansatz die Form der Kalibrierspektren so verändern, als wären sie bereits bei der Zieltemperatur aufgenommen worden. Hierzu sei auf S. Groß, Multivariate Korrektur des Temperatureinflusses in der NIR-spektroskopischen Materialfeuchtebestimmung, Dissertation, Göttingen, 2009 und J.M. Roger et al., EPO-PLS external parameter orthogonalization of PLS application to temperature-independent measurement of sugar content of intact fruits, Chemometrics and intelligent laboratory system, 2003, Vol. 66 (2), p. 191 - 204 verwiesen. Während sich die Arbeit von Groß auf die Feuchtebestimmung von Mehl bei bekannten Temperaturen bezieht, arbeiten Rogers et al. an einem so genannten robusten Modell, in dem die Temperatureinflüsse bereits einbezogen sind, sodass sich eine Messung der Probentemperatur erübrigt. In der US 10 254 215 B2 werden mittels eines handgehaltenen, kompakten Spektrometers durch den Endverbraucher Inhaltsstoffanteile von landwirtschaftlichen Früchten (wie Zuckergehalt von Äpfeln oder Ölgehalt von Avocado) gemessen, wobei eine Temperaturmessung der Probe dazu dient, das gemessene Spektrum in einer nicht näher beschriebenen Weise zu normieren oder zu modifizieren.

Aufgabe

Bei Anwendungen von Spektrometern an landwirtschaftlichen Arbeitsmaschine hat man bisher eine Messung der Temperatur oder einer anderen Eigenschaft der Probe vermieden und zur Auswertung der Spektren die dotierten Kalibrationsmodelle verwendet, in denen die Abhängigkeit der Messung von der Temperatur oder anderen Eigenschaft bereits in den verwendeten Spektren enthalten ist, mit dem erwähnten Nachteil der geringen Genauigkeit.

Die Erfindung hat sich zur Aufgabe gesetzt, die genannten Nachteile zu vermeiden oder zumindest zu vermindern.

Lösung

Diese Aufgabe wird erfindungsgemäß durch die Lehre der Patentansprüche 1 und 6 gelöst, wobei in den weiteren Patentansprüchen Merkmale aufgeführt sind, die die Lösung in vorteilhafter Weise weiterentwickeln.

Eine zur Anbringung an einer landwirtschaftlichen Arbeitsmaschine geeignete Spektrometeranordnung umfasst eine Lichtquelle zur Beleuchtung einer zu untersuchenden Probe, ein Spektrometer zur Bereitstellung eines Spektralsignals hinsichtlich der wellenlängenspezifischen Intensität von der Probe reflektierten und/oder transmittiertem Lichts, und eine Auswertungseinrichtung, die konfiguriert ist, anhand des Spektralsignals des Spektrometers und von abgespeicherten Kalibrierdaten den Gehalt der Probe an einem oder mehreren Inhaltsstoffen zu bestimmen. Der Auswertungseinrichtung ist ein Signal oder sind mehrere Signale hinsichtlich einer oder mehrerer Eigenschaft(en) der Probe zuführbar und die Auswertungseinrichtung ist konfiguriert, das oder die Signal(e) bei der Bestimmung des Gehalts der Probe an dem einen oder mehreren Inhaltsstoffen zu berücksichtigen.

Auf diese Weise vermeidet man die eingangs erwähnten Nachteile.

Das Signal kann durch einen mit der Probe zusammenwirkenden Sensor erzeugbar oder mittels einer Schnittstelle in die Auswertungseinrichtung eingebbar sein. Gemäß der zweiten Variante kann ein Bediener somit die Eigenschaft messen oder von einer anderen Messeinrichtung ablesen oder zumindest schätzen und über die Schnittstelle in die Auswertungseinrichtung eingeben.

Das Signal kann u.a. eine oder mehrere der folgenden Eigenschaften der Probe betreffen: Temperatur, Druck, pH-Wert, Fließgeschwindigkeit und/oder Luftfeuchtigkeit in der Umgebung der Probe etc..

Die Auswertungseinrichtung kann betreibbar sein, anhand des Signals das Spektralsignal zu modifizieren und/oder die Kalibrierdaten zur Bestimmung des Gehalts der Probe an dem einen oder mehreren Inhaltsstoffen zu modifizieren und/oder auszuwählen und/oder den Gehalt der Probe an dem einen oder mehreren Inhaltsstoffen anhand von der Eigenschaft unabhängigen Kalibrierdaten zu bestimmen und diesen anhand der Eigenschaft zu korrigieren. Hierzu sei auf den Stand der Technik nach Groß und Roger et al., a.a.O., sowie die dort erwähnten Literaturstellen verwiesen, die durch Verweis mit in die vorliegenden Unterlagen aufgenommen werden.

Es besteht auch die Möglichkeit, die Kalibrierdaten durch eine selbstlernende Algorithmik (Kalibrierdatenerzeugungseinrichtung) zu erzeugen, z.B. ein neuronales Netzwerk.

Die Spektrometeranordnung kann insbesondere an einer landwirtschaftlichen Arbeitsmaschine verwendet werden, wobei der von der Spektrometeranordnung gemessene Gehalt der Probe, bei der es sich beispielsweise um auf einem Feld stehende Pflanzen oder geerntete Pflanzen oder Gülle handeln kann, an dem einen oder mehreren Inhaltsstoffen zu Dokumentationszwecken georeferenziert abgespeichert und/oder zur Ansteuerung eines Aktors verwendet wird.

Insbesondere kann die Arbeitsmaschine ein Güllefass umfassen und die Spektrometeranordnung mit dem Inhalt des Güllefasses oder der hinein oder daraus fließenden Gülle zusammenwirken, um die Ausbringung der Gülle auf ein Feld basierend auf den durch die Spektrometeranordnung erfassten Gehalt der Probe an dem einem oder mehreren Inhaltsstoffen zu kontrollieren.

Ausführungsbeispiele

In den Zeichnungen sin nachfolgend näher beschriebene Ausführungsbeispiele der Erfindung dargestellt. Es zeigt:

Fig. 1      eine schematische Ansicht einer Spektrometeranordnung,

Fig. 2      ein Flussdiagramm zur Gewinnung der Kalibrierdaten und zur Auswertung der Spektren,

Fig. 3      ein Schema für eine Auswertung der Signale eines Spektrometers mittels eines selbstlernenden Systems unter Verwendung eines neuronalen Netzes,

Fig. 4      eine seitliche Ansicht einer landwirtschaftlichen Arbeitsmaschine in Form eines Güllefasses mit einer Spektrometeranordnung, und

Fig. 5    eine seitliche Ansicht einer landwirtschaftlichen Arbeitsmaschine in Form eines Mähdreschers mit einer Spektrometeranordnung.

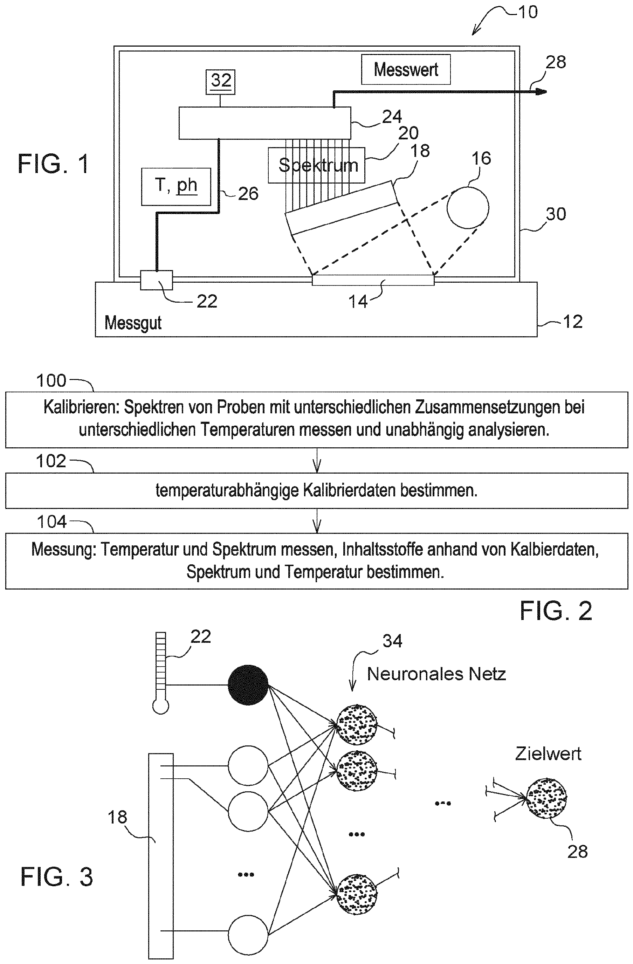

Die Figur 1 zeigt schematisch eine Spektrometeranordnung 10 zur Erfassung des Gehalts einer Probe 12 an einem oder mehreren Inhaltsstoffen. Innerhalb eines Gehäuses 30 ist ein Fenster 14 angeordnet, durch das eine Lichtquelle 16 die außerhalb des Gehäuses 30 liegende Probe 12 mit Licht beaufschlagt. Das Licht kann breitbandig sein oder nur diskrete Wellenlängen umfassen, wobei die Wellenlänge üblicherweise den nahen Infrarotbereich umfasst. Die Probe 12 kann gegenüber dem Gehäuse 30 stationär sein oder sich gegenüber dem Gehäuse 30 bewegen, z.B. daran als Erntegutstrom in einer Erntemaschine oder als Güllestrom in einem Güllefass bei dessen Befüllen oder beim Ausbringen vorbeigefördert werden, oder umgekehrt, indem das Gehäuse 30 an der Probe 12 entlang bewegt wird, indem es z.B. an einem Schlitten befestigt ist, der über ein auf einem Feld liegendes Schwad geführt wird.

Von der Probe 12 reflektiertes (oder im Fall, dass die Lichtquelle 16 außerhalb des Gehäuses 30 angeordnet ist, durch die Probe transmittiertes) Licht wird durch ein im Wellenlängenbereich der Lichtquelle 16 sensitives Spektrometer 18 analysiert, das ein Spektralsignal 20 abgibt, dass für bestimmte Wellenlängen des empfangenen Lichts die zugehörige Intensität angibt. Das Spektrometer 18 kann in an sich bekannter Weise ein dispersives Element (z.B. Prisma, Filter, Gitter etc.) umfassen, welches das einlaufende Licht in von der Wellenlänge abhängige Richtungen ablenkt, das von mehreren lichtempfindlichen, jeweils einem Wellenlängenbereich zugeordneten Elementen (Fotodioden, CCDs o.dgl.) empfangen wird (s. DE 199 22 867 C5 und den dort zitierten Stand der Technik). Bei einer anderen Ausführungsform ist nur ein einziges lichtempfindliches Element vorhanden, dem das Licht unterschiedlicher Wellenlängen zeitlich nacheinander zugeführt wird (vgl. EP 3 444 577 A1). Das Spektralsignal 20 enthält somit eine Information hinsichtlich der Intensitäten unterschiedlicher Wellenlängen des von der Probe 12 reflektierten und/oder transmittierten Lichts, das von der Lichtquelle 16 bereitgestellt wurde.

Eine Auswertungseinrichtung 24, die auch außerhalb des Gehäuses 30 angeordnet sein könnte, erhält das Spektralsignal 20 sowie ein Signal 26, das von einem mit der Probe 12 zusammenwirkenden Sensor 22 bereitgestellt wird, und berechnet im Betrieb anhand des Spektralsignals 20 und des Signals 26 ein Ausgangssignal 28, das den Gehalt der Probe 12 an einem oder mehreren Inhaltsstoffen repräsentiert. Hierbei werden Kalibrierdaten verwendet, die in einem Speicher 32 abgelegt sind. Der Sensor 22 erfasst die Temperatur und/oder den pH-Wert der Probe 12, oder eine beliebige andere Eigenschaft, wie z.B. den Druck der Probe, ihre Bewegungsgeschwindigkeit gegenüber dem Gehäuse 30 und/oder die Luftfeuchtigkeit. Der Sensor 22 könnte auch im Abstand vom Gehäuse 30 angeordnet sein oder durch eine Schnittstelle ersetzt oder ergänzt werden, mit welcher ein Bediener einen geeigneten Wert eingeben kann, sei er geschätzt oder mittels eines anderen Sensors gemessen oder als Prozesstemperatur o.ä. bekannt. Das Signal des Sensors 22 dient dazu, die Abhängigkeit der von der Probe 12 erfassten Spektren von der Eigenschaft, die durch den Sensor 22 erfasst wird, auszugleichen. Das kann auf eine der im Folgenden näher beschriebenen Vorgehensweisen erfolgen.

Die Figur 2 zeigt ein Flussdiagramm, nach welchem die Kalibrierdaten gewonnen und bei der Auswertung der Spektren berücksichtigt werden können.

Im Schritt 100 erfolgt zunächst eine Referenzmessung an einer hinreichend großen Anzahl an Proben 12, wozu eine oder mehrere Spektrometeranordnungen 10 verwendet werden können, wie sie in der Figur 1 gezeigt sind. Hierbei handelt es sich in der Regel um gleichartige Spektrometeranordnungen 10, wie sie auch bei der späteren Messungen verwendet werden, aber nicht um dieselben Spektrometeranordnungen 10. Üblicherweise erfolgt der Schritt 100 mit mehreren Spektrometeranordnungen 10 gleichzeitig oder nacheinander, um Streuungen der individuellen Spektrometeranordnungen 10 ausgleichen zu können. Es werden somit Spektren anhand einer Anzahl an Proben 12 bei unterschiedlichen, bekannten Temperaturen erfasst und mit den zugehörigen Temperaturen abgespeichert. D.h. durch die Spektrometeranordnung 10 werden an einer Probe gleichzeitig das Spektrum und die zugehörigen Randparameter, z.B. die Temperatur, gemessen. Zudem erfolgt eine Analyse der Proben auf bestimmte Inhaltsstoffe in einem Labor oder einem beliebigen, anderen Ort anhand an sich bekannter, chemischer oder physikalischer Verfahren.

Nach dem Schritt 100 stehen somit die Spektren, zugehörige (gemessene oder auf andere Weise z.B. als Prozessparameter bekannt gewordene) Temperaturen und bekannte Inhaltsstoffgehalte der Proben zur Verfügung. Anhand dieser Daten werden im Schritt 102 die Kalibrierdaten bestimmt, die im Speicher 32 abgelegt werden.

Im Schritt 104 erfolgt dann die Messung mit der in der Figur 1 gezeigten Spektrometeranordnung 10. Anhand der mit dem Sensor 22 erfassten Temperatur (und/oder des pH-Wertes oder einer oder mehrerer der anderen, oben erwähnten Eigenschaften, der oder die anstelle oder zusätzlich zur Temperatur in den Schritten 100 und 102 mit in die Kalibrierdaten eingeflossen sein kann oder können) und des Spektralsignals 20 wird der jeweilige Inhaltsstoff bestimmt und als Ausgangssignal 28 zur weiteren Verwendung bereitgestellt, z.B. zu Dokumentationszwecken oder zur Kontrolle eines Aktors.

Im Einzelnen kann die Berücksichtigung der Temperatur und/oder des pH-Werts und/oder einer oder mehrerer der anderen Eigenschaften im Schritt 104 in der Weise erfolgen, dass zunächst das Spektralsignal 20 einer Vorverarbeitung unterzogen wird und in bisher üblicher Weise eine von der Temperatur und/oder dem pH-Wert unabhängige Evaluierung des Inhaltsstoffgehalts der Probe anhand der im Speicher 32 abgelegten Kalibrierdaten erfolgt. Dieser unkorrigierte Inhaltsstoffgehalt kann anschließend anhand der Temperatur und/oder einer anderen, oben erwähnten Eigenschaft korrigiert werden, wozu man beispielsweise einen linearen oder einen beliebigen, anderen Zusammenhang verwenden kann. Eine andere Möglichkeit besteht darin, für unterschiedliche Temperaturen und/oder Eigenschaften unterschiedliche Kalibrierdaten zu verwenden und anhand der Temperatur und/oder Eigenschaft aus dem Speicher 32 abzurufen. Auch besteht die Möglichkeit, das Spektralsignal 20 vor der Auswertung anhand der Temperatur und/oder Eigenschaft zu modifizieren und letztlich einer in bisher üblicher Weise von der Temperatur und/oder dem pH-Wert unabhängigen Evaluierung des Inhaltsstoffgehalts der Probe anhand der im Speicher 32 abgelegter Kalibrierdaten zu unterziehen.

Ein Regressionsmodell für das Spektrometer 18 mit n Kanälen in der Form $i = \sum_n x_i c_i + c_0$, basierend auf einem Kalibrierdatensatz mit m Proben kann beispielsweise nach dem Prinzip der kleinsten Quadrate erstellt werden. Hier bezeichnet $i$ einen Inhaltstoffwert, z.B. die Materialfeuchte, $c_i$ einen Satz von n Regressionskoeffizienten, $c_0$ einen Offsetparameter und $x_i$ sind die Messwerte des Spektrometers die das gemessene Spektrum darstellen. Dazu werden die Sensormesswerte in einer n x m Matrix $x$ zusammengefasst (Sensormesswerte pro Probe stehen in den Zeilen) und die n Koeffizienten $\vec{c}$. Zusätzlich wird eine Spalte mit 1 (geschrieben als $\vec{1}$)aufgenommen um einen Offsetparameter in das Modell einzufügen:

$$\left[\vec{1}, \underline{x}\right]_{m\,x\,(n+1)} \left(1,\vec{c}\right)_{n+1} = \vec{i}_m$$

Sei im Folgenden $\underline{X} = [\vec{1}, \underline{x}]_{mx(n+1)}$ und $\vec{C} = (1, \vec{c})_{n+1}$. Dann können die Regressionskoeffizienten mit der Pseudoinversen direkt berechnet werden:

$$\vec{C} = \left(\underline{X}^T\underline{X}\right)^{-1} \underline{X}^T \vec{i}_m$$

Diese Berechnung ist im Allgemeinen nicht auf Spektralsensoren übertragbar, da die Kanäle des Spektrometers 18 untereinander hohe Korrelationen aufweisen. Daher werden in der Regel Verfahren der Principle Component oder Partial Least Square Regression angewendet.

Bei der Principle Component Regression wir die Matrix x zunächst einer Hauptachsentransformation unterzogen. Als neue Basisvektoren zur Beschreibung der Sensormesswerte verwendet man die Eigenvektoren der Kovarianzmatrix $K = cov(\underline{x}, \underline{x})$, auch Moden, Hauptkomponenten oder Loadings genannt. Das bedeutet, die Matrix $\underline{x}$ wird überführt in eine transformierte Matrix $\underline{s} = \underline{L}^{-1}\underline{x}^T$, wobei die Transformationsmatrix $\underline{L}^{-1}$ aus den Loadings und $\underline{S}$ ist die X entsprechende Matrix im neuen Koordinatensystem und analog zu oben $\underline{S} = [\vec{1}, \underline{s}]$. Die Koordinaten im transformierten Koordinatensystem $\underline{s}$ werden üblicherweise Scores genannt.

Im neuen System kann dann ein Regressionsmodell erstellt werden durch:

$$\vec{c}_s = \left(\underline{S}^T\underline{S}\right)^{-1} \underline{S}^T \vec{i}_m$$

Die resultierenden Regressionskoeffizienten können im Ausgangssystem beschrieben werden, indem eine Rücktransformation $\vec{c} = \underline{L}\,\vec{c}_s$ ausgeführt wird.

Im Kalibrierprozess wird die Regression nur mit wenigen der n Hauptkomponenten durchgeführt. Mit der Wahl der Hauptkomponenten lässt sich die Modellkomplexität kontrollieren.

Werden Messwerte ($t_i$) eines zusätzlichen Sensors 22 im Kalibrierdatensatz erhoben und sollen diese in der Regression berücksichtigt werden, können sie in einer Variante zu den Sensorwerten gruppiert werden $\underline{X} = [\vec{1}, \vec{t}, \underline{x}]_{mx(n+2)}$ und das Modell wird um einen Koeffizienten für den Temperaturmesswert erweitert $(1,\vec{c})_{n+1} \rightarrow (1, c_t\vec{c})_{n+2}$. Danach werden die Hauptachsentransformation und die nachfolgende Regression auf den Scores wie zuvor ausgeführt.

Alternativ können die Messwerte $t_i$ des Sensors 22 erst nach der Transformation zu den Scores hinzugruppiert werden, d.h. $\underline{S}$ wird erweitert zu $[\vec{t}\ \underline{S}]$, und die Temperaturwerte werden wie eine der Hauptkomponenten behandelt und mit einem Koeffizient $c_s^t$ in ins Modell aufgenommen, also $\vec{c}_s \rightarrow (c_s^t, \vec{c}_s)$. Die Rücktransformation wird dann nur auf den Messwerten des Spektrometers 18 ausgeführt.

Alternativ können auch separate Regressionsmodelle wie oben beschrieben für verschiedene Temperaturbereiche berechnet werden und dann mit Hilfe des Temperatursensors wird das jeweils passende Modell für die entsprechende Materialtemperatur ausgewählt werden.

Zur Vorhersage kann auch ein nicht lineares Modell verwendet werden, das den Messwert des Sensors 26 zur Vorhersage des Inhaltsstoffs verwendet. Besonders vielversprechend erscheint der Ansatz, wenn zur Vorhersage ein fortschrittlicheres Verfahren des maschinellen (Selbst-) Lernens, wie z.B. ein neuronales Netz 34, verwendet wird, das als Eingangswerte die spektralen Messungen des Spektrometers 18 und die Messwerte des Sensors 26 verwendet. Das bedeutet, die Messwerte des Sensors 26 werden als Eingang in ein z.B. in ein neuronales Netz 34 aufgenommen (Fig 3). Das Netz 34 wird dann mit den Messwerten des Spektrometers 18 und denen des unabhängigen (Temperatur-) Sensors 22 trainiert und im Betrieb wiederum die Messwerte des Sensors 22 und des Spektrometers 18 verwendet.

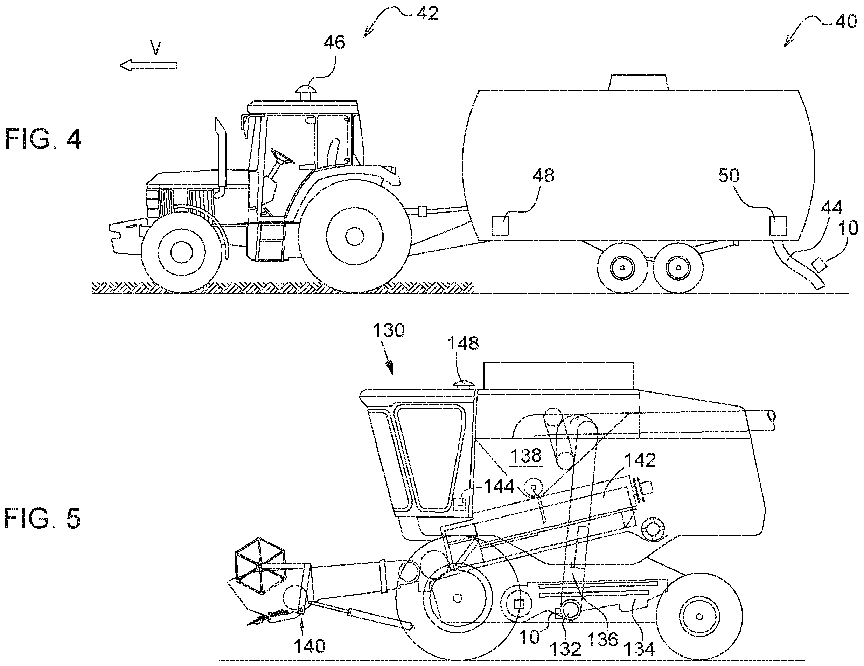

In der Figur 4 ist eine landwirtschaftliche Arbeitsmaschine 40 in Form eines Güllefasses dargestellt, das durch einen Ackerschlepper 42 über ein Feld gezogen wird, um Gülle durch eine Leitung 44 auf ein Feld auszubringen. Das Ausgangssignal 28 der Spektrometeranordnung 10, die mit der in der Leitung 44 fließenden Gülle zusammenwirkt, wird einer Steuerung 48 zugeführt, die zudem mit einem Positionsbestimmungssystem 46 verbunden ist. Die Steuerung 48 kontrolliert ein Ventil 50, das die Flussrate der Gülle durch die Leitung 44 bestimmt, basierend auf einer in der Steuerung 48 im vorab abgespeicherten Ausbringkarte, in welcher festgelegt wird, wieviel Inhaltstoff (z.B. Stickstoff, Kalium etc.) je Flächeneinheit ausgebracht werden soll, und anhand des Ausgangssignals 28 im Sinne einer Einhaltung der in der Ausbringkarte festgelegten Werte. Der Sensor 22 der Spektrometeranordnung 10 erfasst bei dieser Ausführungsform insbesondere die Temperatur der Probe 12, optional auch deren Druck und/oder pH-Wert. Seine Ausgangswerte dienen zur Korrektur des Ausgangssignals 28 in einer der oben beschriebenen Weisen.

Die Figur 5 zeigt eine weitere Ausführungsform einer Arbeitsmaschine 130 in Form eines selbstfahrenden Mähdreschers 130, die mit einer Spektrometeranordnung 10 ausgestattet ist. Die Spektrometeranordnung 10 ist an der Wand einer Querförderschnecke 132 angebracht und wirkt durch eine Öffnung in der Wand mit dem gereinigten Erntegut (Körnern) zusammen, das von einer Reinigungseinrichtung 134 abgegeben und durch die Querförderschnecke 132 einem Körnerelevator 136 übergeben wird, der es in einem Korntank 138 ablegt. Die Reinigungseinrichtung 134 erhält das Erntegut von einer Erntegutaufnahmeeinrichtung 140, die es einer Dresch- und Trenneinheit 142 zuführt. Die Spektrometeranordnung 10 liefert bestimmte Inhaltsstoffangaben wie Proteingehalt, Stärkegehalt, Ölgehalt und Feuchtigkeitsanteil des geernteten Korns, die durch eine Kontrolleinheit 144 gemeinsam mit einer Positionsinformation, die von einer Empfangsantenne 148 eines satellitenbasierten Positionsbestimmungssystems bereitgestellt wird, in einer Karte abgespeichert werden. Der Sensor 22 erfasst bei dieser Ausführungsform insbesondere die Temperatur der Probe 12, optional auch die Luftfeuchtigkeit in der Umgebung der Arbeitsmaschine 130 und/oder die Fließgeschwindigkeit des sensierten Korns. Seine Ausgangswerte dienen zur Korrektur des Ausgangssignals 28, insbesondere hinsichtlich des Feuchtigkeitsgehalts des Ernteguts, in einer der oben beschriebenen Weisen.

**Patentansprüche**

1. Spektrometeranordnung (10) für eine landwirtschaftliche Arbeitsmaschine (40, 130), umfassend:

   eine Lichtquelle (16) zur Beleuchtung einer zu untersuchenden Probe (12),
   ein Spektrometer (18) zur Bereitstellung eines Spektralsignals (20) hinsichtlich der wellenlängenspezifischen Intensität von der Probe (12) reflektierten und/oder transmittiertem Lichts, und
   einer Auswertungseinrichtung (24), die konfiguriert ist, anhand des Spektralsignals (22) des Spektrometers (18) und von abgespeicherten Kalibrierdaten den Gehalt der Probe (12) an einem oder mehreren Inhaltsstoffen zu bestimmen,
   **dadurch gekennzeichnet, dass** der Auswertungseinrichtung (24) ein Signal (26) hinsichtlich einer Eigenschaft der Probe (12) zuführbar ist und die Auswertungseinrichtung (24) konfiguriert ist, das Signal (26) bei der Bestimmung des Gehalts der Probe (12) an dem einen oder mehreren Inhaltsstoffen zu berücksichtigen.

2. Spektrometeranordnung (10) nach Anspruch 1, wobei das Signal (26) durch einen mit der Probe zusammenwirkenden Sensor (22) erzeugbar oder mittels einer Schnittstelle in die Auswertungseinrichtung (24) eingebbar ist.

3. Spektrometeranordnung (10) nach Anspruch 1 oder 2, wobei das Signal (26) eine oder mehrere folgender Eigenschaften der Probe (12) betrifft: Temperatur, Druck, pH-Wert, Fließgeschwindigkeit und/oder Luftfeuchtigkeit in der Umgebung der Probe.

4. Spektrometeranordnung (10) nach einem der vorhergehenden Ansprüche, wobei die Auswertungseinrichtung (24) konfiguriert ist, anhand des Signals (26) das Spektralsignal (20) zu modifizieren und/oder die Kalibrierdaten zur Bestimmung des Gehalts der Probe (26) an dem einen oder mehreren Inhaltsstoffen zu modifizieren und/oder auszuwählen und/oder den Gehalt der Probe (12) an dem einen oder mehreren Inhaltsstoffen anhand von der Eigenschaft unabhängigen Kalibrierdaten zu bestimmen und diesen ermittelten Gehalt anhand der Eigenschaft zu korrigieren.

5. Spekrometeranordnung (10) nach einem der vorhergehenden Ansprüche, wobei die Kalibrierdaten durch eine selbstlernende Kalibrierdatenerzeugungseinrichtung erzeugt sind.

6. Spektrometeranordnung (10) nach Anspruch 5, wobei die Auswertungseinrichtung (24) ein neuronales Netzwerk (34) umfasst.

7. Landwirtschaftliche Arbeitsmaschine (40, 130) mit einer Spektrometeranordnung (10) nach einem der vorhergehenden Ansprüche.

8. Arbeitsmaschine (40, 130) nach Anspruch 7, wobei der von der Spektrometeranordnung (10) gemessene Gehalt der Probe (12) an dem einen oder mehreren Inhaltsstoffen zu Dokumentationszwecken georeferenziert abgespeichert und/oder zur Ansteuerung eines Aktors verwendet wird.

9. Arbeitsmaschine (40) nach Anspruch 7 oder 8, wobei die Arbeitsmaschine ein Güllefass umfasst und die Spektrometeranordnung (10) mit dem Inhalt des Güllefasses oder der hinein oder daraus fließenden Gülle zusammenwirkt, um die Ausbringung der Gülle auf ein Feld basierend auf den durch die Spektrometeranordnung (10) erfassten Gehalt der Probe (12) an dem einem oder mehreren Inhaltsstoffen zu kontrollieren.

**FIG. 1**

**FIG. 2**

| |
|---|
| 100 — |
| Kalibrieren: Spektren von Proben mit unterschiedlichen Zusammensetzungen bei unterschiedlichen Temperaturen messen und unabhängig analysieren. |
| 102 — |
| temperaturabhängige Kalibrierdaten bestimmen. |
| 104 — |
| Messung: Temperatur und Spektrum messen, Inhaltsstoffe anhand von Kalbierdaten, Spektrum und Temperatur bestimmen. |

**FIG. 3**

FIG. 4

FIG. 5

EP 3 865 855 A1

Europäisches Patentamt
European Patent Office
Office européen des brevets

# EUROPÄISCHER RECHERCHENBERICHT

**Nummer der Anmeldung**

EP 21 15 1863

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | XIU YING LIANG ET AL: "Study of sample temperature compensation in the measurement of soil moisture content", MEASUREMENT, INSTITUTE OF MEASUREMENT AND CONTROL. LONDON, GB, Bd. 44, Nr. 10, 6. Juli 2011 (2011-07-06), Seiten 2200-2204, XP028320223, ISSN: 0263-2241, DOI: 10.1016/J.MEASUREMENT.2011.07.008 [gefunden am 2011-07-14] | 1-6 | INV. G01N21/27 G01N21/31 G01N21/84 |
| Y | * das ganze Dokument * ----- | 7-9 | |
| Y | WO 98/21930 A1 (CASE CORP [US]) 28. Mai 1998 (1998-05-28) * Seite 3, Absatz 2 * * Seite 5 * ----- | 7-9 | |
| Y | NL 1 015 440 C2 (VMA VLASTUIN MEST APPLICATIES) 17. Dezember 2001 (2001-12-17) * Ansprüche 1,3 * & Vma Vlastuin Mest Applicaties: "English Translation of NL1015440C2", , 17. Dezember 2001 (2001-12-17), Seiten 1-5, XP055815455, Gefunden im Internet: URL:https://patents.google.com/patent/NL10 15440C2/en?oq=+NL1015440C2 [gefunden am 2021-06-18] * Seite 4, Absatz 5 * ----- | 9 | |
| A | DE 10 2017 220103 A1 (FRAUNHOFER GES FORSCHUNG [DE]; KARLSRUHER INSTITUT FUER TECH KIT [DE]) 16. Mai 2019 (2019-05-16) * Absatz [0002] * ----- -/-- | 1-9 | |

**RECHERCHIERTE SACHGEBIETE (IPC)**

G01N

Der vorliegende Recherchenbericht wurde für alle Patentansprüche erstellt

| Recherchenort | Abschlußdatum der Recherche | Prüfer |
|---|---|---|
| München | 18. Juni 2021 | Huenges, Alexandra |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

Europäisches Patentamt

European Patent Office

Office européen des brevets

**EUROPÄISCHER RECHERCHENBERICHT**

Nummer der Anmeldung

EP 21 15 1863

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | CHAUCHARD J M ET AL: "Correction of the temperature effect on near infrared calibration - application to soluble solid content prediction", J. NEAR INFRARED SPECTROSC, Bd. 12, 1. Januar 2004 (2004-01-01), Seiten 199-205, XP055813422, * Zusammenfassung * | 1-9 | |

----- 

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 18. Juni 2021 | Huenges, Alexandra |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.........................................................................
& : Mitglied der gleichen Patentfamilie, übereinstimmendes Dokument

EPO FORM 1503 03.82 (P04C03)

Seite 2 von 2

**ANHANG ZUM EUROPÄISCHEN RECHERCHENBERICHT
ÜBER DIE EUROPÄISCHE PATENTANMELDUNG NR.**

EP 21 15 1863

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-06-2021

| Im Recherchenbericht angeführtes Patentdokument | Datum der Veröffentlichung | Mitglied(er) der Patentfamilie | Datum der Veröffentlichung |
|---|---|---|---|
| WO 9821930 A1 | 28-05-1998 | AR 011017 A1<br>AU 5443598 A<br>US 6029106 A<br>WO 9821930 A1 | 02-08-2000<br>10-06-1998<br>22-02-2000<br>28-05-1998 |
| NL 1015440 C2 | 17-12-2001 | KEINE | |
| DE 102017220103 A1 | 16-05-2019 | DE 102017220103 A1<br>EP 3707496 A1<br>US 2021181093 A1<br>WO 2019092147 A1 | 16-05-2019<br>16-09-2020<br>17-06-2021<br>16-05-2019 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005083386 A1
- US 10254215 B2
- DE 19922867 C5
- EP 3444577 A1


**In der Beschreibung aufgeführte Nicht-Patentliteratur**

- **J.B. REEVES III et al.** Near-Infrared Spectroscopic Determination of Carbon, Total Nitrogen, and Ammonium-N in Dairy Manures. *J Dairy Sci,* 2000, vol. 83, 1829-1836
- **C. PAUL et al.** Influence of sample temperature on the assessment of quality characteristics in undried forages by Near Infrared Spectroscopy (NIRS). *Landbauforschung Völkenrode,* April 2002, 229-237
- **PEINADO et al.** Temperature-induced variation for NIR tensor-based calibration. *Chemometrics and Intelligent Laboratory Systems,* 2006, vol. 83, 75-82
- **J.M. ROGER et al.** EPO-PLS external parameter orthogonalization of PLS application to temperature-independent measurement of sugar content of intact fruits. *Chemometrics and intelligent laboratory system,* 2003, vol. 66 (2), 191-204